(19) Europäisches Patentamt
European Patent Office
Office européen des brevets

(11) EP 4 736 767 A1

(12) EUROPEAN PATENT APPLICATION
published in accordance with Art. 153(4) EPC

(43) Date of publication:
06.05.2026 Bulletin 2026/19

(21) Application number: 24851833.4

(22) Date of filing: 06.08.2024

(51) International Patent Classification (IPC):
A61B 5/332 (2021.01)        A61B 5/256 (2021.01)
A61B 5/352 (2021.01)        A61B 5/353 (2021.01)

(52) Cooperative Patent Classification (CPC):
A61B 5/256; A61B 5/332; A61B 5/352; A61B 5/353

(86) International application number:
PCT/JP2024/028011

(87) International publication number:
WO 2025/033409 (13.02.2025 Gazette 2025/07)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 09.08.2023 JP 2023130328

(71) Applicants:
• OMRON Corporation
Kyoto-shi, Kyoto 600-8530 (JP)
• Omron Healthcare Co., Ltd.
Muko-shi, Kyoto 617-0002 (JP)

(72) Inventors:
• KIMURA ISHIDA, Yui
Kyoto-shi, Kyoto 600-8530 (JP)

• KOIZUMI, Masayuki
Kyoto-shi, Kyoto 600-8530 (JP)
• WANG, Danni
Kyoto-shi, Kyoto 600-8530 (JP)
• KUBO, Mitsuaki
Kyoto-shi, Kyoto 600-8530 (JP)
• KAWABATA, Yasuhiro
Muko-shi, Kyoto 617-0002 (JP)
• FUJII, Kenji
Muko-shi, Kyoto 617-0002 (JP)
• MATSUMURA, Naomi
Muko-shi, Kyoto 617-0002 (JP)
• FUKUNAGA, Seiji
Muko-shi, Kyoto 617-0002 (JP)
• YOSHIDA, Takuya
Kyoto-shi, Kyoto 600-8234 (JP)

(74) Representative: Isarpatent
Patent- und Rechtsanwälte Part G mbB
Friedrichstrasse 31
80801 München (DE)

(54) **BIOLOGICAL SIGNAL MEASUREMENT DEVICE**

(57) A biological signal measurement device that is wearable, capable of measuring biological signals from a plurality of channels, and driven by a battery includes: an operation mode switching unit configured to switch between a first operation mode in which the plurality of channels are activated and biological signals from the plurality of channels are measured and a second operation mode in which only some of the channels are activated and biological signals from some of the channels are measured; a remaining battery level acquisition unit configured to acquire remaining battery level information of the battery; a prediction unit configured to predict an operable time of the biological signal measurement device based on power consumption in each operation mode and the remaining battery level; and an output unit configured to output information about a predicted value of an operable time.

FIG. 1

## Description

TECHNICAL FIELD

The present invention relates to a wearable biological signal measurement device.

BACKGROUND OF INVENTION

Wearable biological signal measurement devices worn on a body continuously for a long period of time to monitor biological signals are known. For example, there are a variety of products available, ranging from medical devices such as 24-hour Holter electrocardiograph monitors to consumer devices such as smart watches and life loggers. This type of device has a limitation that it needs to be removed from the body when charging the built-in battery, and therefore biological signals cannot be measured while charging. Although it is not necessary to continue measuring for 24 hours, there is a need to reduce the time when measurement is not possible due to charging and the number of times charging is required as much as possible. It is undesirable for measurements to be interrupted for a long period of time due to a dead battery.

Patent Document 1 discloses a wearable electrocardiograph that is worn on the upper arm to measure electrocardiogram (ECG) signals. This electrocardiograph is also driven by a built-in battery, but the abovementioned charging issue is not particularly taken into consideration.

CITATION LIST

PATENT LITERATURE

Patent Document 1: WO 2005/027720

SUMMARY

TECHNICAL PROBLEM

Some conventional products have a function that shows the remaining battery level as a percentage or an icon. However, even if a user knows the remaining battery level, it is difficult for the user to predict how many more hours the device can be used (how long the device can continue measuring), making it difficult to determine the appropriate timing to charge it.

As the present inventors continue to develop a measurement device having a performance capable of measuring biological signals from a plurality of channels, they are studying dynamically switching the number of active channels (channels performing measurements) depending on the quality of the signals being measured and the user's condition. In the case of such measurement devices, power consumption varies depending on the number of active channels, which raises concerns that it may become even more difficult for users to determine the appropriate timing for charging on their own.

The present invention has been made in view of the above circumstances, and an object of the present invention is to provide information to inform a user of an appropriate timing for charging in a wearable biological signal measurement device that can dynamically switch the number of channels to be activated.

SOLUTION TO PROBLEM

An embodiment of the present disclosure is a biological signal measurement device that is wearable, capable of measuring biological signals from a plurality of channels, and driven by a battery and includes: an operation mode switching unit configured to switch between a first operation mode in which the plurality of channels are activated and biological signals from the plurality of channels are measured and a second operation mode in which only some of the channels are activated and biological signals from some of the channels are measured; a remaining battery level acquisition unit configured to acquire remaining battery level information of the battery; a prediction unit configured to predict an operable time of the biological signal measurement device based on power consumption in each operation mode and the remaining battery level; and an output unit configured to output information about a predicted value of an operable time.

The prediction unit may predict a first operable time when the biological signal measurement device continues operating in the first operation mode based on the power consumption in the first operation mode and the remaining battery level and predict a second operable time when the biological signal measurement device continues operating in the second operation mode based on the power consumption in the second operation mode and the remaining battery level. The output unit may output the first operable time and the second operable time as minimum and maximum values, respectively, of the predicted value of the operable time.

The output unit may determine whether charging is necessary based on the predicted value of the operable time and notify a user that charging is necessary when charging is necessary.

The output unit may control a timing of a notification such that the notification is provided during a time period in which the user is able to charge the battery.

The output unit may consider that the user has returned home when a current time passes a preset time, and provide the notification.

The output unit may determine an at-home time period in which the user is at home based on schedule information of the user acquired from an external device, and provide the notification during the at-home time period.

The output unit may estimate a time period in which the user is able to charge the battery based on charging history information that records a time when charging has been performed in the past, and provide the notification during the time period estimated to be available for charging.

The output unit may determine whether the user is at home based on location information of the user, and provide the notification when it is determined that the user is at home.

The output unit may notify the user that charging is necessary using at least one of sound, vibration, light, and electrical stimulation.

The output unit may include a notification device that notifies the user that charging is necessary using light, and the notification device may be disposed at a position visible to the user wearing the biological signal measurement device.

The output unit may acquire information about a current time and information about a chargeable time period in which the user is expected to be able to charge the battery the following day, and determine that charging is necessary during the current day when an expected operation stop time of the biological signal measurement device, which is calculated from the current time and the operable time, arrives before the chargeable time period on the following day.

The prediction unit may predict a proportion of a future operation time in each of the first operation mode and the second operation mode, and calculate the operable time based on the proportion of the future operation time in each operation mode, the power consumption in each operation mode, and the remaining battery level.

The prediction unit may analyze time series data of biological signals measured in the past and predict a proportion of a future operation time in each operation mode based on a result of an analysis.

The prediction unit may perform: analyzing a state of a waveform of the time series data of the biological signals measured in the past to divide the time series data into a clean section in which a quality of the waveform is equal to or higher than a predetermined criterion and a noisy section in which the quality of the waveform is lower than the predetermined criterion; predicting a proportion of a future operation time in the first operation mode from a proportion of the noisy section; and predicting a proportion of a future operation time in the second operation mode from a proportion of the clean section.

The prediction unit may perform: analyzing a state of a waveform of the time series data of the biological signals measured in the past to divide the time series data into a clean section in which a quality of the waveform is equal to or higher than a predetermined criterion and a noisy section in which the quality of the waveform is lower than the predetermined criterion, and further dividing the clean section into an abnormal waveform section in which an abnormal waveform appears and a normal waveform section in which the abnormal waveform does not appear; predicting a proportion of a future operation time in the first operation mode from a proportion of the noisy section and the abnormal waveform section; and predicting a proportion of a future operation time in the second operation mode from a proportion of the normal waveform section.

The biological signal may be an electrocardiogram (ECG) signal.

The present invention may be regarded as a biological signal measurement device including at least a part of the above configuration, or may be regarded as an electrocardiogram measurement device that measures an ECG signal as a biological signal. The present invention can also be understood as a charging information providing method including at least a part of the above processing, a control method for a biological signal measurement device, or a program for implementing such a method or a recording medium on which such a program is non-temporarily recorded. The present invention can be configured by combining each of the above-described configurations and processes to the extent possible.

ADVANTAGEOUS EFFECTS OF INVENTION

According to the present invention, it is possible to provide information to inform a user of an appropriate timing for charging in a wearable biological signal measurement device that can dynamically switch the number of active channels.

BRIEF DESCRIPTION OF THE DRAWINGS

[FIG. 1] FIG. 1 is a diagram illustrating a state in which a biological signal measurement device is worn on an upper arm.
[FIG. 2] FIG. 2 is a plan view of the biological signal measurement device.
[FIG. 3] FIG. 3 is a perspective view of the biological signal measurement device.
[FIG. 4] FIG. 4 is a block diagram illustrating a hardware configuration of the biological signal measurement device.
[FIG. 5] FIG. 5 is a block diagram illustrating a functional configuration of a control body.
[FIG. 6] FIG. 6 is a flowchart illustrating a flow of a measurement process of an ECG signal.
[FIG. 7] FIG. 7 is a diagram illustrating examples of operation modes and active channels.
[FIG. 8] FIG. 8 is a diagram illustrating a waveform of an ECG signal and heart rate information.
[FIG. 9] FIG. 9 is a diagram illustrating a relationship between signal quality, an operation mode, and power consumption.
[FIG. 10] FIG. 10 is a flowchart illustrating a flow of a prediction process of an operable time in a first embodiment.
[FIG. 11] FIG. 11 is a diagram illustrating an example of an operable time display screen.

[FIG. 12] FIG. 12 is a flowchart illustrating a flow of a prediction process of an operable time in a second embodiment.

[FIG. 13] FIG. 13 is a flowchart illustrating a flow of a notification process in a third embodiment.

[FIG. 14] FIG. 14 is a flowchart illustrating a flow of a notification process in a fourth embodiment.

DESCRIPTION OF EMBODIMENTS

<Application Example>

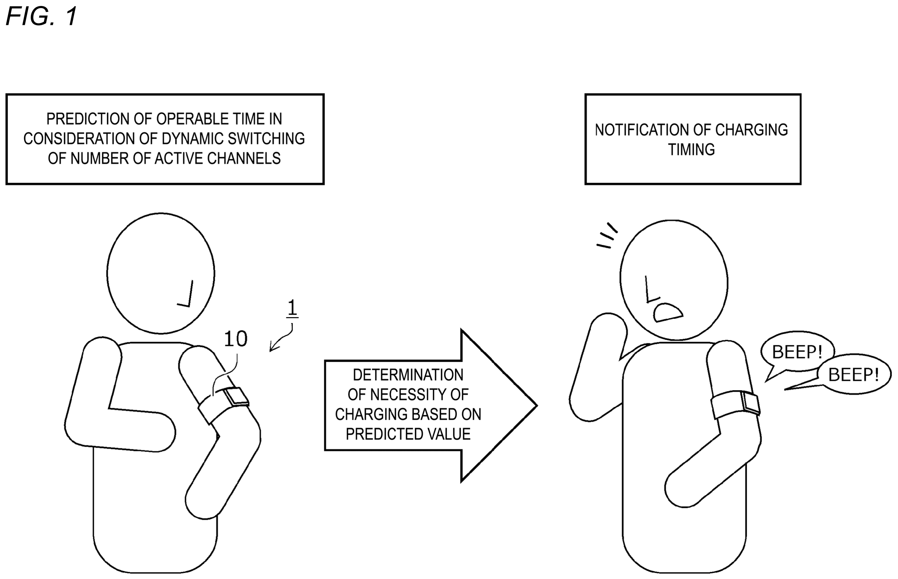

An application example of the present invention will be described with reference to FIG. 1.

A biological signal measurement device 1 is a wearable measurement device capable of measuring a biological signal of a user. Examples of biological signals that can be measured include an electrocardiogram signal (a minute electrical signal associated with a heartbeat), an electromyographic signal (a minute electrical signal associated with muscle activity), an electroencephalogram signal (a minute electrical signal associated with brain activity), pulse waves, blood pressures, and the like.

At the time of measurement, a user wears the biological signal measurement device 1 by wrapping a band 10 around a measurement site. Examples of measurement sites where the band 10 is attached include upper limbs (upper arms, forearms, wrists, hands, and fingers), lower limbs (thighs, lower legs, ankles, feet, and toes), head, neck, chest, abdomen, and earlobe, and a measurement site is appropriately selected depending on the type of biological signal to be measured, a measurement algorithm, and the like.

The biological signal measurement device 1 has an operation mode switching function that can switch between a first operation mode in which a plurality of channels are activated and biological signals from a plurality of channels are measured and a second operation mode in which only some of the channels are activated and biological signals from some of the channels are measured. For example, when highly accurate or reliable measurements are required, the device may operate in the first operation mode to collect as much data as possible, and when measurements with sufficient accuracy are possible even with a small number of channels, the device may operate in the second operation mode to reduce power consumption.

In the most basic configuration, the first operation mode is a mode in which all channels are used (also called a multi-channel mode), and the second operation mode is a mode in which only one channel is used (also called a single-channel mode). However, the present invention is not limited to this configuration, and the number of channels to be activated in each operation mode may be set arbitrarily. For example, when the total number of channels possessed by the biological signal measurement device 1 is N, the number of active channels in the first operation mode is n1, and the number of active channels in the second operation mode is n2, it is sufficient that $N \geq n1 > n2 \geq 1$. Third, fourth, etc. operation modes may be provided which have different numbers of active channels from the first and second operation modes.

The biological signal measurement device 1 is configured to operate continuously for several days to several weeks with the battery fully charged, and basically, it is assumed that the biological signal measurement device 1 is always worn by the user to perform 24-hour monitoring of biological signals. Since the biological signal measurement device 1 must be removed from the body only when the battery is being charged, it is desirable to charge the device as few times as possible in order to minimize gaps in the measurement period. However, if the user refrains from charging and the battery runs out while the user is out or at work and unable to charge the battery, the user will not be able to resume measuring until the user returns home and charges the battery, which will result in a long gap in measurement time. Some common wearable devices show the remaining battery level as a percentage or an icon, but it is difficult to ascertain how much longer measurement time remains (that is, how much longer one does not need to charge the battery) using percentage or icon displays. In particular, in the case of a device that can dynamically switch operation modes as in the present invention, the amount of power consumed will vary depending on how the operation mode is subsequently switched, making it difficult for the user to determine the appropriate timing for charging on their own.

Therefore, the biological signal measurement device 1 predicts the operable time of the biological signal measurement device 1 based on the power consumption in each operation mode and the remaining battery level, and outputs information about the predicted value of the operable time. Furthermore, the device may determine whether charging is necessary based on the predicted value of the operable time, and when charging is necessary, notify the user that charging is necessary at an appropriate timing using means such as sound, vibration, light, or electrical stimulation.

For example, the biological signal measurement device 1 may predict a first operable time when the device continues operating in the first operation mode based on the power consumption and the remaining battery level in the first operation mode, and may also predict a second operable time when the device continues to operate in the second operation mode based on the power consumption and remaining battery level in the second operation mode. The first operable time corresponds to the operable time when the power consumption is estimated to be the highest, and the second operable time corresponds to the operable time when the power consumption is estimated to be the lowest. If the first operable time and the second operable time are presented as the minimum and maximum values, respectively, of the predicted value of the operable time, the user knows that charging only needs to be performed within the time range of the presented predicted values, making it easier for the user to plan when to charge.

Alternatively, the biological signal measurement device 1 may predict the proportion of the future operation time in each of the first operation mode and the second operation mode, and calculate the operable time based on the proportion of the future operation time in each operation mode, the power consumption in each operation mode, and the remaining battery level. By predicting the proportion of the future operation time in each operation mode and estimating the operable time, it is possible to present the operable time taking into account actual use. Accordingly, it is possible for the user to determine the appropriate timing for charging. The operation time proportion may be predicted by any method. For example, time series data of biological signals measured in the past may be analyzed, and a proportion of a future operation time in each operation mode may be predicted based on a result of an analysis. By analyzing data measured in the past, it is possible to ascertain how each operation mode was switched during the actual use of the biological signal measurement device 1 by the user, thereby making it possible to estimate the operable time with high accuracy.

Hereinafter, as an embodiment of the present invention, a specific configuration example in a case where the present invention is applied to an upper arm electrocardiographic device will be described.

<First Embodiment>

(Device Configuration)

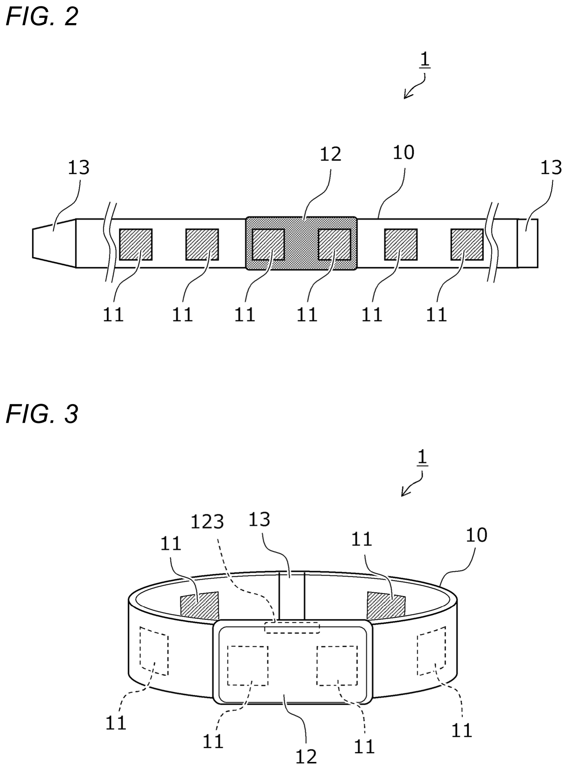

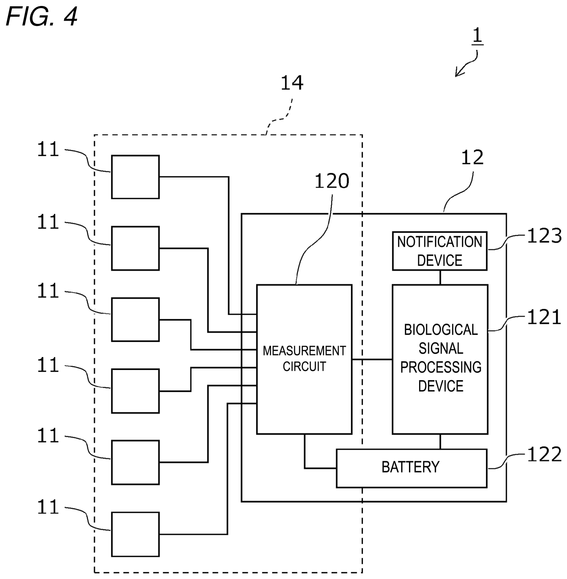

An embodiment of the present invention will be described with reference to FIGS. 1 to 4. FIG. 1 is a diagram illustrating a state in which the biological signal measurement device 1 is worn on an upper arm, FIG. 2 is a plan view of the biological signal measurement device 1, FIG. 3 is a perspective view of the biological signal measurement device 1, and FIG. 4 is a block diagram illustrating the hardware configuration of the biological signal measurement device 1.

The biological signal measurement device 1 of the present embodiment is an upper arm electrocardiographic device that is worn on an upper arm (preferably, a left upper arm close to the heart) of a user and is used to measure an electrocardiogram (ECG) signal as a biological signal.

The biological signal measurement device 1 includes, as main components, a band 10, a plurality of electrodes 11 fixed to the band 10, and a control body 12 fixed to the band 10.

The band 10 is a member (fixing member) for fixing the electrodes 11 in a state in which the electrodes are pressed against the living body. In the present embodiment, a belt-shaped band 10 made of a flexible and pliable material (for example, chemical fiber, silicon, leather, or the like) is used. A fixing mechanism 13 is provided at a longitudinal end portion of the band 10. As illustrated in FIGS. 1 and 3, the biological signal measurement device 1 can be worn on the upper arm by forming the band 10 into a loop shape and fastening the band with the fixing mechanism 13. The fixing mechanism 13 may be any mechanism such as a hook-and-loop fastener, a hook, a connector, a button, or a magnet.

The plurality of electrodes 11 (also referred to as an electrode array) are embedded and fixed in the band 10 such that a contact surface with the living body is exposed to an inner side (living body side) of the band 10. The plurality of electrodes 11 are arranged in a line at equal intervals in the longitudinal direction of the band 10. Thus, when the band 10 is wrapped around the arm, the electrodes 11 come into contact with different circumferential positions of the arm. The number of electrodes 11 can be appropriately designed. At least two electrodes 11 (a pair of electrodes) may be provided to measure an ECG signal, and three or more electrodes 11 may be provided to increase the reliability and robustness of measurement. In the present embodiment, a configuration in which six electrodes 11 (three electrode pairs) are provided is employed.

The electrode 11 is a dry-type metal electrode. The wet-type electrode (gel electrode or the like) has problems such as a possibility of causing skin rash or itching when attached for a long time, and low durability and maintainability, whereas the dry-type electrode 11 does not have such problems. The biological signal measurement device 1 of the present embodiment is assumed to be worn continuously for a long time and to monitor the ECG signal for 24 hours, and thus the electrodes 11 are preferably dry-type electrodes.

The control body 12 is a processing unit that performs control and signal processing of the biological signal measurement device 1. As illustrated in FIG. 4, the control body 12 has a structure in which a measurement circuit 120 for measuring ECG signals, a biological signal processing device 121 for performing various signal processing on the ECG signals, a battery 122, a notification device 123, and other circuits are mounted inside a case made of resin or metal, for example. The control body 12 may be provided with a physical switch and a display. The measurement circuit 120 is an analog circuit configured with a differential amplifier and the like, and the plurality of electrodes 11 and the measurement circuit 120 constitute an ECG sensor 14. The biological signal processing device 121 is configured with, for example, an AD converter, a filter, a processor, a memory, and the like. The battery 122 is a rechargeable secondary battery. The AD converter, the filter, the processor, the memory, and other circuits included in the biological signal processing device 121 are driven by power from the battery 122. The notification device 123 is a device that notifies the user that charging is necessary and may be any type of device, such as a buzzer or a speaker that emits sound, a vibrator that emits vibration, a light source that emits light, or an electrode that emits electrical stimulation. Furthermore, a plurality of types of notification devices may be provided. When using light for notification, it is advisable to dispose the light source at a position where the light can be seen by the user wearing the biological signal measurement device 1, for example, on the upper side of the control body 12 (the side facing the base of the arm) as illustrated in FIG. 3.

(Control Body)

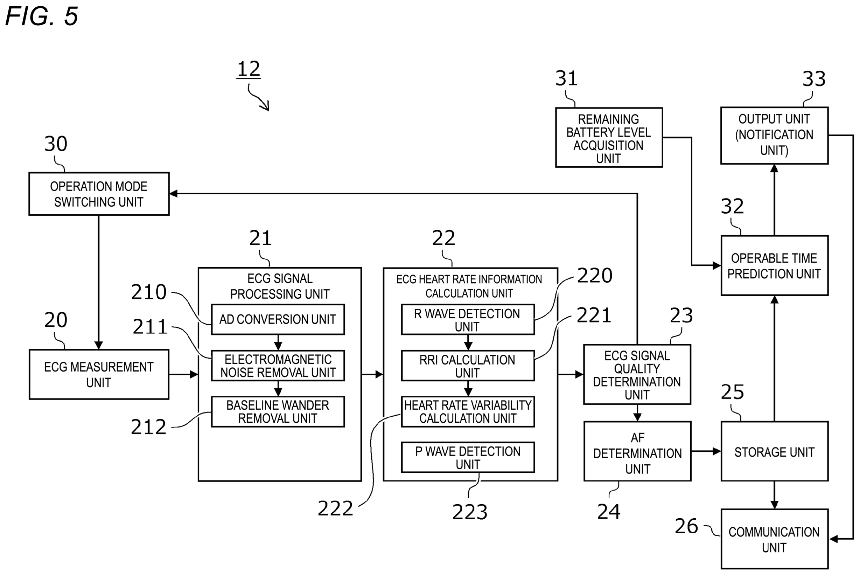

FIG. 5 is a block diagram illustrating an example of a functional configuration of the control body 12.

The control body 12 includes, as components related to the measurement of the ECG signal, an operation mode switching unit 30, an ECG measurement unit 20, an ECG signal processing unit 21, an ECG heart rate information calculation unit 22, an ECG signal quality determination unit 23, an AF determination unit 24, a storage unit 25, and a communication unit 26. The operation mode switching unit 30 is a part that dynamically switches the number of active channels used for measurement according to the quality of the ECG signal to be measured. The ECG measurement unit 20 is a part that amplifies the potential difference between the active electrode pair using a differential amplifier and outputs the amplified potential difference as an ECG signal. The ECG signal processing unit 21 is a part that performs AD conversion and filtering process of the ECG signal, and includes an AD conversion unit 210 that performs analog-to-digital conversion of the ECG signal using an AD converter, an electromagnetic noise removal unit 211 that removes electromagnetic noise from the ECG signal to increase the S/N ratio, and a baseline wander removal unit 212 that removes baseline wander (low-frequency fluctuations) from the ECG signal. The ECG heart rate information calculation unit 22 is a part that extracts various types of heart rate information from the ECG signal, and includes an R wave detection unit 220, an RRI calculation unit 221, a heart rate variability calculation unit 222, and a P wave detection unit 223. The ECG signal quality determination unit 23 is a part that determines whether the quality of the ECG signal is good or poor (that is, whether accurate or reliable data suitable for diagnosis or the like can be measured). The AF determination unit 24 is a part that detects an occurrence of Atrial Fibrillation (AF) based on the heart rate information and calculates an indicator related to AF. The storage unit 25 is a non-volatile memory that stores measured or calculated data. The storage unit 25 also stores parameters (such as the power consumption value for each operation mode) that are referenced when predicting the operable time, which will be described later. The communication unit 26 is a part that performs wireless data communication with an external device (for example, a smartphone of a user, another health device, a home server, or the like).

like).

The control body 12 also includes a remaining battery level acquisition unit 31, an operable time prediction unit 32, and an output unit (notification unit) 33 as components related to prediction of the operable time. The remaining battery level acquisition unit 31 is a part configured to acquire information about the remaining level of the battery. The operable time prediction unit 32 is a part configured to predict the operable time of the biological signal measurement device 1 based on the power consumption in each operation mode and the remaining battery level. The output unit (notification unit) 33 is a part that outputs information about the predicted value of the operable time, determines whether charging is necessary based on the predicted value of the operable time, and notifies the user that charging is necessary at an appropriate timing when charging is necessary.

Among the functional units illustrated in FIG. 5, the ECG measurement unit 20 is a function provided by the measurement circuit 120 in FIG. 4, the output unit 33 is a function provided by the cooperation of the biological signal processing device 121 and the notification device 123 in FIG. 4, and the other functional units are functions provided by the biological signal processing device 121 in FIG. 4.

(Measurement Process of ECG Signal)

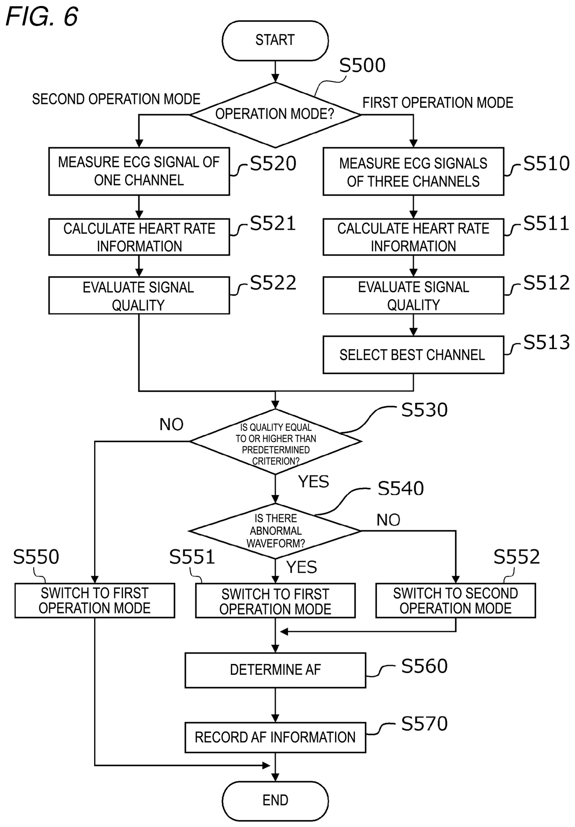

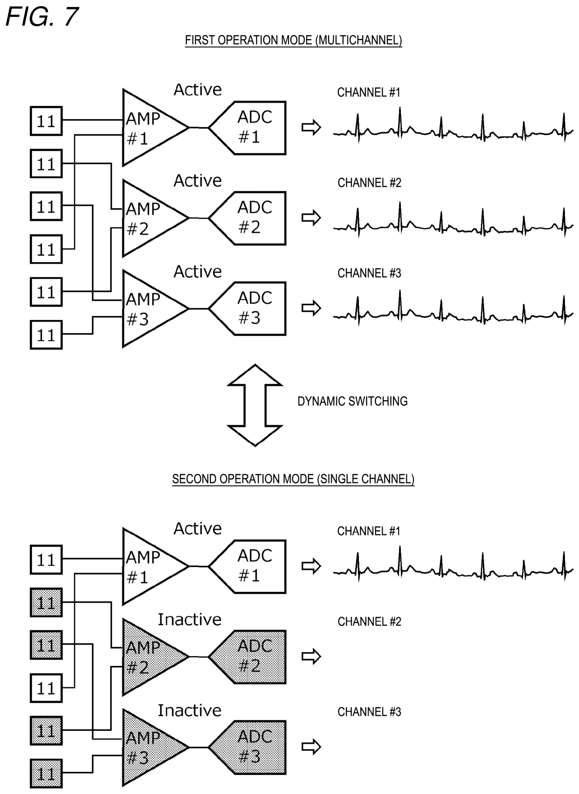

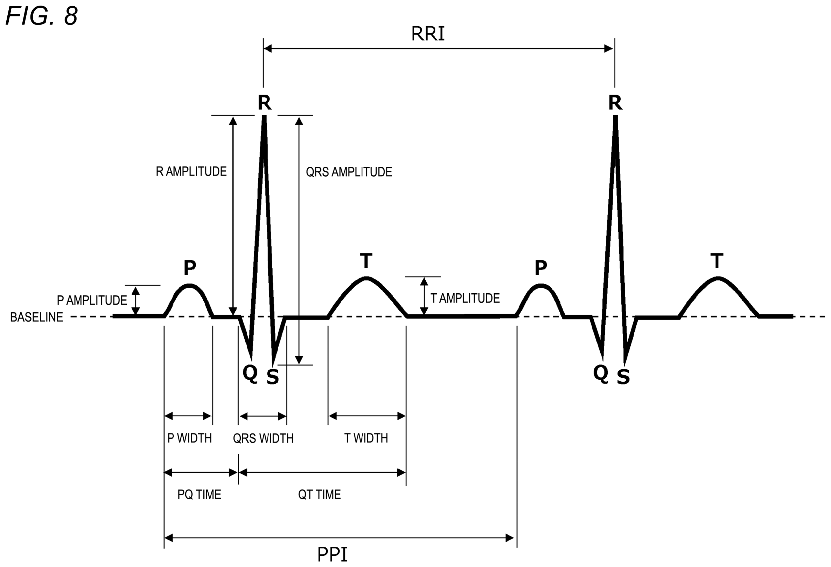

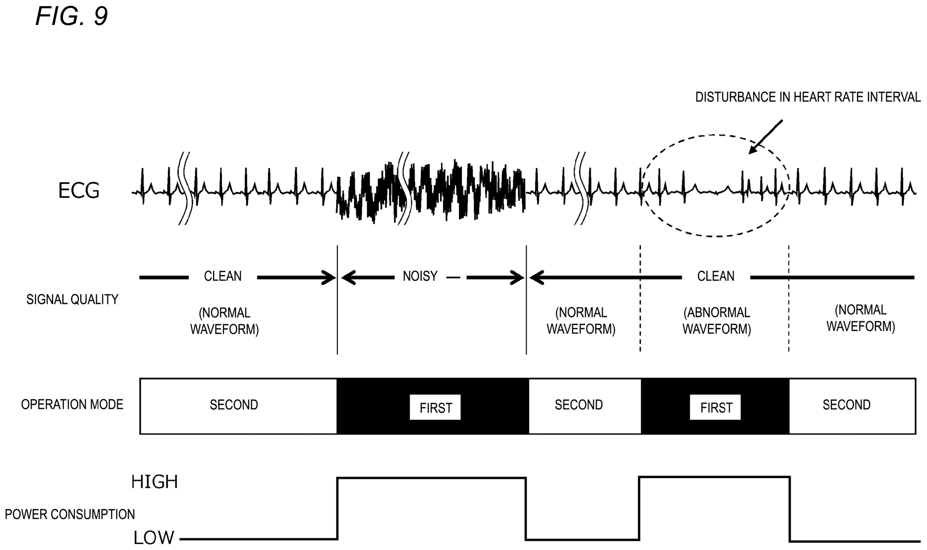

The measurement process of an ECG signal by the biological signal measurement device 1 will be described with reference to FIGS. 6 to 9. FIG. 6 is a flowchart illustrating a flow of the measurement process of an ECG signal, FIG. 7 is a diagram illustrating examples of operation modes and active channels, FIG. 8 is a diagram illustrating a waveform of an ECG signal and heart rate information, and FIG. 9 is a diagram illustrating a relationship between signal quality, an operation mode, and power consumption.

When the user wraps the band 10 around the upper arm, fastens the band 10 with the fixing mechanism 13, and then performs an operation to instruct the start of measurement, the processor of the control body 12 starts the measurement process illustrated in FIG. 6.

In step S500, the operation mode switching unit 30 selects a channel to be activated in accordance with the current operation mode. As illustrated in FIG. 7, the biological signal measurement device 1 of the present embodiment has a first operation mode (multi-channel mode) in which all three electrode pairs and the corresponding circuits (such as differential amplifiers AMP#1 to AMP#3 and AD converters ADC#1 to ADC#3) are activated and ECG signals of three channels #1 to #3 are measured and a second operation mode (single-channel mode) in which only one electrode pair and the corresponding circuit (in the example of FIG. 7, differential amplifier AMP#1 and AD converter ADC#1) are activated and only an ECG signal of one channel is measured.

In the present embodiment, as illustrated in FIG. 7, three electrode pairs are set such that two electrodes 11 that exactly face each other when the band 10 is wrapped around the upper arm form a pair. This is because the greater the distance between the two electrodes 11 forming a pair, the greater the potential difference (that is, an ECG signal with a high S/N ratio) that can be measured. The distance between the two electrodes 11 is the linear distance between the two electrodes 11 when measured with the band 10 wrapped around the upper arm as illustrated in FIG. 3, for example. However, the method of setting the electrode pairs is not limited thereto. For example, a multiplexer may be used to freely switch the combination of the electrodes 11 to be paired. In this case, ECG signals of four or more channels can be measured from the six electrodes 11.

(Case of First Operation Mode)

In step S510, the ECG measurement unit 20 measures ECG signals of three channels using three electrode pairs. Specifically, power is supplied from the battery 122 to each of the three differential amplifiers AMP#1 to AMP#3 and the three AD converters ADC#1 to ADC#3 to activate them (to a driven state), and the signals of each of the three electrode pairs are input to the corresponding differential amplifiers AMP#1 to AMP#3. The potential difference between the two electrodes 11 that constitute the electrode pair is amplified by a differential amplifier and output as an ECG signal (analog voltage signal).

The ECG signals of three channels (analog voltage signals) output from the differential amplifiers AMP#1 to AMP#3 are analog-to-digital converted by the corresponding AD converters ADC#1 to ADC#3 and output as ECG signals of three channels (digital signals). The ECG signal of each channel is subjected to digital signal processing to remove electromagnetic noise and baseline wander. A known noise reduction method such as a band-pass filter, a notch filter, or a moving average can be used to remove electromagnetic noise and baseline wander.

In step S511, the ECG heart rate information calculation unit 22 analyzes the three-channel ECG signals that have been acquired and calculates various types of heart rate information. The ECG heart rate information calculation unit 22 executes the process of step S511 when time series data (waveform data) of the ECG signal for a predetermined unit time is taken in from the ECG signal processing unit 21. The unit time may be set to, for example, about 10 seconds to 600 seconds, and in the present embodiment is set to 10 seconds.

As schematically illustrated in FIG. 8, the waveform of one heart rate of an ECG signal mainly consists of a P wave, a QRS wave, and a T wave. The QRS wave indicates a waveform obtained by combining an R wave, which is an upward peak, and a downward Q wave and a downward S wave that appear before and after the R wave. In a normal heart, an electrical stimulus generated at the sinoatrial node is transmitted from the atrium to the ventricle, and excitation (contraction) of the atrium and excitation (contraction) of the ventricle occur in sequence, thereby pumping blood. The P wave is a waveform corresponding to the excitation of the atrium, the QRS wave is a waveform corresponding to the excitation of the ventricle, and the T wave is a waveform corresponding to the process of recovery of the ventricle from the excitation. In the case of an average adult, the heart rate is about 60 to 80 bpm, such that the time series data of an ECG signal for a unit time (10 seconds) includes about 10 to 14 heart rate waveforms.

Examples of the heart rate information include an R amplitude (height of R wave from the baseline), a QRS amplitude (defined by, for example, the sum of the height of R wave from the baseline and the depth of S wave from the baseline), a P amplitude (height of P wave from the baseline), a T amplitude (height of T wave from the baseline), a P width (time from start of P wave to end of P wave), a QRS width (time from start of Q wave to end of S wave), a T width (time from start of T wave to end of T wave), a PQ time (time from start of P wave to start of Q wave), a QT time (time from start of Q wave to end of T wave), an RRI (RR interval; time from peak of R wave to peak of next R wave), a PPI (PP interval; time from start of P wave to start of next P wave), a heart rate variability (temporal variation in RRI and PPI), and the like. It is not necessary to calculate all of the heart rate information, and only necessary heart rate information may be calculated. Other heart rate information may be calculated.

In step S512, the ECG signal quality determination unit 23 evaluates the quality of the ECG signal for the unit time taken-in in step S510. Any indicator may be used for the evaluation of the signal quality. For example, the noise, power (intensity), baseline wander, etc. of the ECG signal itself may be used as the evaluation index, or the heart rate information calculated in step S511 (including whether the calculation of the heart rate information was successful) may be used as the evaluation index. In step S512, the evaluation indices for the ECG signals of the three channels are calculated.

In step S513, the ECG signal quality determination unit 23 selects a channel from which the ECG signal with the best quality (the ECG signal with the highest evaluation index value) is obtained from among the three channels. The ECG signal of the channel selected here is used in subsequent processing.

In this way, in the first operation mode, ECG signals from a plurality of channels are measured redundantly, and the ECG signal with the best quality is selected from among them and used for processing such as AF determination. Therefore, measurements that are robust against influences such as body movement can be achieved.

(Case of Second Operation Mode)

In step S520, the ECG measurement unit 20 measures only ECG signals of one channel using one electrode pair. For example, in the example of FIG. 7, power is supplied from the battery 122 to one differential amplifier AMP#1 and one AD converter ADC#1, and power supply to other differential amplifiers AMP#2 and AMP#3 and other AD converters ADC#2 and ADC#3 is stopped. Accordingly, only one channel #1 becomes active (in a driven state). The channel to be activated is not limited to #1, but channel #2 or channel #3 may be activated. The potential difference between one electrode pair corresponding to an active channel is amplified by a differential amplifier and output as an ECG signal (analog voltage signal) of one channel. The ECG signal of one channel output from the differential amplifier is analog-to-digital converted by a corresponding AD converter and output as an ECG signal (digital signal) of one channel. The ECG signal of one channel is subjected to digital signal processing to remove electromagnetic noise and baseline wander. In step S521, the ECG heart rate information calculation unit 22 analyzes the taken-in ECG signal of one channel and calculates various types of heart rate information. In step S522, the ECG signal quality determination unit 23 evaluates the quality of the ECG signal for the unit time taken-in in step S520. The processing in steps S520 to S522 and the processing in steps S510 to S512 are the same except for the number of channels to be processed.

In step S530, the ECG signal quality determination unit 23 determines whether the quality of the most recently measured ECG signal for the unit time (the ECG signal selected in step S513 in the case of the first operation mode) satisfies a predetermined criterion. The predetermined criterion is a threshold value for determining whether the ECG signal is accurate or reliable data suitable for purposes such as diagnosis. For example, the predetermined criterion may be whether a heart rate waveform can be identified from an ECG signal (whether R waves and P waves have been successfully detected). Hereinafter, an ECG signal determined to have a quality equal to or higher than a predetermined criterion will be referred to as a "clean signal", and an ECG signal determined not to satisfy the predetermined criterion will be referred to as a "noisy signal".

When the ECG signal is determined to be clean (YES in step S530), the ECG signal quality determination unit 23 determines whether an abnormal waveform is included in the ECG signal for this unit time (step S540). For example, when the heart rate interval (RRI) is constant, it may be determined that "there is no abnormal waveform", and when the heart rate interval is random, it may be determined that "there is an abnormal waveform".

The results of the determinations made in steps S530 and S540 are sent to the operation mode switching unit 30. When the most recently measured ECG signal is determined to be noisy (NO in step S530), the operation mode switching unit 30 switches the operation mode to the first operation mode (step S550). When the ECG signal is determined to be clean but to have an abnormal waveform (YES in step S540), the operation mode switching unit 30 also switches the operation mode to the first operation mode (step S551). Then, when the ECG signal is determined to be clean and to have no abnormal waveform (NO in step S540), the operation mode switching unit 30 switches the operation mode to the second operation mode (step S552).

In step S560, the AF determination unit 24 determines whether AF (atrial fibrillation) is occurring based on information about the presence or absence of P waves and information about a heart rate variability. For example, when the information indicates that "there is no P wave" and that "heart rate variability is present", it is determined that atrial fibrillation has occurred during the unit time indicated by the waveform data. Although not illustrated, at the timing when the occurrence of atrial fibrillation is detected, the output unit (notification unit) 33 may notify the user that charging is necessary, or an alert message or the like may be output from the communication unit 26 to an external device.

In step S570, the AF determination unit 24 records, in the storage unit 25, the presence or absence of occurrence of atrial fibrillation together with information about the measurement time of the waveform data. At this time, the waveform data and the heart rate information calculated in steps S511 and S521 may be recorded together. Accordingly, the AF determination results for each unit time are accumulated in the storage unit 25.

The measurement process illustrated in FIG. 6 described above is repeatedly executed every unit time (for example, every 10 seconds), thereby dynamically switching the operation mode (number of active channels) depending on the quality and state of the measured signal, and performing 24-hour monitoring of the ECG signal.

FIG. 9 illustrates the relationship between the quality of the ECG signal, the operation mode, and the power consumption.

For example, when the S/N ratio of the ECG signal is reduced due to the influence of myoelectric noise caused by body movement, the signal quality is determined to be noisy. In the noisy section, multi-channel measurements are performed in the first operation mode. By performing redundant measurements using electrode pairs disposed in different locations and selecting the best signal from these to use for diagnosis, it is expected that the stability and reliability of measurements and diagnoses can be improved.

On the other hand, in a clean section in which an ECG signal with a clean waveform is measured, measurement is performed in a single channel in the second operation mode in principle. In the second operation mode, power supply to circuits not used for measurement (such as differential amplifiers, AD converters, filters, etc.) is stopped, thereby reducing the power consumed by the circuits compared to the first operation mode. In the second operation mode, since it is only necessary to perform processes such as calculating heart rate information (S521 in FIG. 6) and evaluating the quality of the ECG signal (S522 in FIG. 6) on ECG signals for one channel, the power consumed by the processor can be reduced compared to the first operation mode, which requires processing of ECG signals for three channels. The measurement process in FIG. 6 is repeatedly executed at regular unit time intervals (for example, 10 seconds) regardless of whether the operation mode is the first operation mode or the second operation mode. Therefore, during the clean period when the ECG signal is stable (that is, the period of operation in the second operation mode), the amount of power consumption per unit time can be reduced compared to the noisy period (that is, the period of operation in the first operation mode).

However, even in a clean section, when an abnormal waveform such as a disturbance in the heart rate interval appears, the operation mode is immediately switched to the first operation mode, and multi-channel measurement is performed. This enables more accurate capture of signal waveforms in situations where arrhythmia is occurring, enabling highly accurate and reliable diagnosis. For example, in the AF determination in step S560, in order to accurately determine whether atrial fibrillation is occurring, it is necessary to check the disturbance in the heart rate interval and the presence or absence of P waves. This is because it is not possible to distinguish whether the arrhythmia is due to atrial fibrillation or another disease (such as premature ventricular contractions) based solely on the disturbance in the heart rate interval. The presence or absence of P waves (waves indicating excitation of the atria) is evaluated, and a diagnosis of atrial fibrillation can only be made once it is confirmed that P waves are not being formed. However, as illustrated in FIG. 8, since the amplitude of the P wave is small, it may be difficult to identify the P wave even in a clean signal. If ECG signals from a plurality of channels are used, it is expected that P waves can be identified, that is, the presence or absence of P waves can be determined with high accuracy, and as a result, the accuracy of AF determination can be improved.

When a disturbance in the heart rate interval is detected during single-channel measurement in the second operation mode, it may not be possible to accurately detect P waves from the waveform data for that unit time, making it impossible to confirm whether atrial fibrillation is occurring. However, it is considered sufficient to switch to the first operation mode when a disturbance in the heart rate interval is detected and to perform multi-channel measurement from the next unit time. There is a concern that AF detection will be delayed by approximately one unit time (for example, 10 seconds), but since AF is considered to be high risk when it continues for 30 seconds or more, this amount of time lag is practically acceptable.

(Prediction of Operable Time)

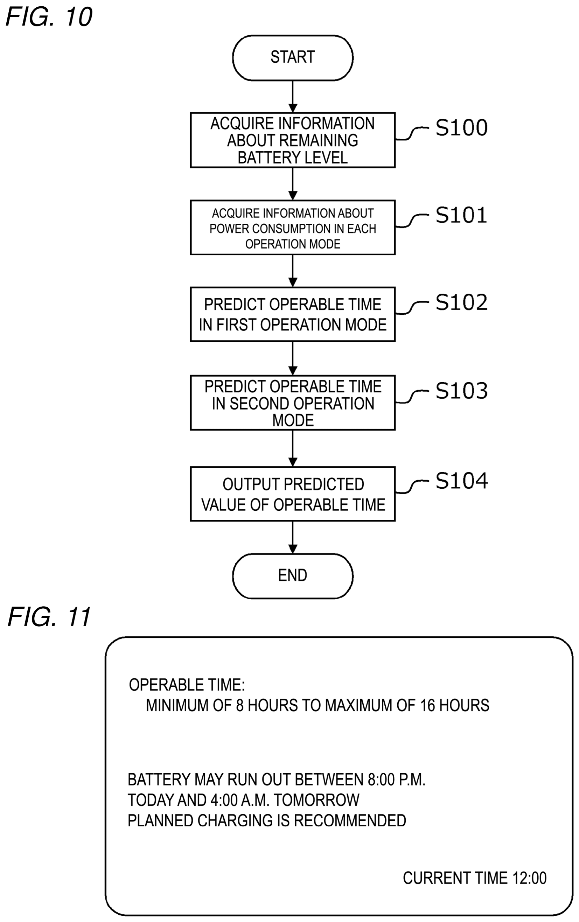

FIG. 10 illustrates a flow of a prediction process of an operable time in a first embodiment. The prediction process of FIG. 10 is executed at predetermined time intervals (for example, at a frequency once every few minutes to every few tens of minutes).

In step S100, the remaining battery level acquisition unit 31 acquires information about a remaining battery level RM of the biological signal measurement device 1. The remaining battery level can be measured using, for example, a gas gauge circuit.

In step S101, the operable time prediction unit 32 reads information about power consumption PC1 in the first operation mode and power consumption PC2 in the second operation mode from the storage unit 25. The power consumption value for each operation mode is assumed to be obtained by experiment or the like and written in advance in the storage unit 25 before the biological signal measurement device 1 is shipped from the factory.

In step S102, the operable time prediction unit 32 predicts a first operable time AT1 when the biological signal measurement device 1 continues operating in the first operation mode from the power consumption PC1 in the first operation mode and the remaining battery level RM. The simplest prediction formula is

$$AT1 \text{ [h]} = RM \text{ [W·h]} / PC1 \text{ [W]}$$

However, the operable time AT1 may be calculated using an advanced prediction formula that takes into account various variable factors, without being limited to this prediction formula.

In step S103, the operable time prediction unit 32 predicts a second operable time AT2 when the biological signal measurement device 1 continues operating in the second operation mode from the power consumption PC2 in the second operation mode and the remaining battery level RM. The simplest prediction formula is

$$AT2 \text{ [h]} = RM \text{ [W·h]} / PC2 \text{ [W]}$$

However, the operable time AT2 may be calculated using an advanced prediction formula that takes into account various variable factors, without being limited to this prediction formula.

Since

$$PC1 > PC2, AT1 < AT2.$$

In step S104, the output unit 33 displays the information about AT1 and AT2 on a display, or notifies an external device (such as the user's smartphone) via the communication unit 26. FIG. 11 is an example of an operable time display screen displayed on the biological signal measurement device 1 or an external device. AT1 is displayed as the minimum value of the predicted value of the operable time, and AT2 is displayed as the maximum value of the predicted value of the operable time. Furthermore, as illustrated in FIG. 11, the time when the battery will run out may be calculated from the current time and the predicted value of the operable time, and the time (that is, the time when charging is required) may be notified to the user. When notifying the user of the predicted value of the operable time or the time when charging is required, values that take into account a small margin of time may be notified such that charging can be performed before the battery runs out completely.

As illustrated in FIG. 11, the predicted values for the operable time and the time when charging is required are presented as a time range (minimum value to maximum value), such that the user can roughly ascertain during what period charging should be performed, making it easier to establish a charging plan. In the example of FIG. 11, when the remaining operable time is displayed as a minimum of 8 hours to a maximum of 16 hours as of the current time of 12 o'clock, the user will know that the battery will run out between 8:00 p.m. during the current day and 4:00 a.m., such that the user can make a plan according to their own schedule and lifestyle, such as, "When I get home during the current day, I'll remove device 1 and charge it while I'm preparing dinner or while I'm taking a bath".

The prediction process of the present embodiment described above can predict the operable times AT1 and AT2, with the power consumption PC1 and PC2 for each operation mode taken into account, and provide information to inform the user of the appropriate timing for charging in the wearable biological signal measurement device 1 that can dynamically switch the number of channels to be activated. This improves the usability of the biological signal measurement device 1 and enables 24-hour monitoring with minimal gaps in measurement.

<Second Embodiment>

In the prediction process of the first embodiment, the operable time was predicted individually for each operation mode, whereas in a prediction process of a second embodiment, the proportion of the future operation time in each of the first operation mode and the second operation mode is predicted, and the operable time is calculated based on the proportion of the future operation time in each operation mode, the power consumption in each operation mode, and the remaining battery level.

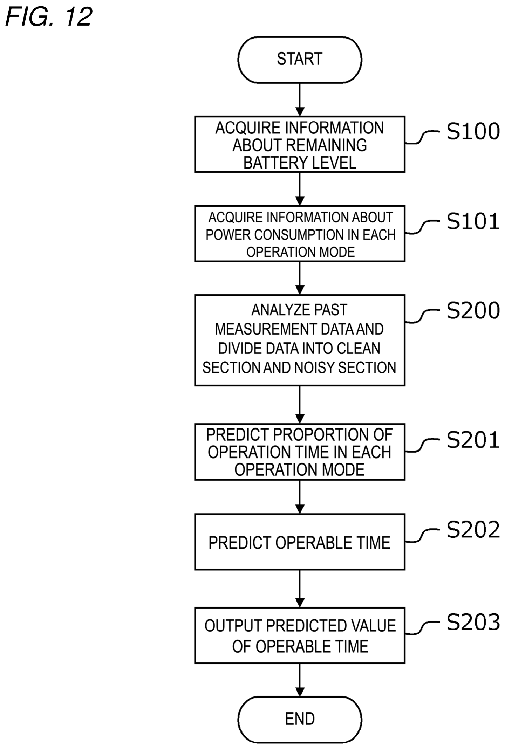

FIG. 12 illustrates a flow of a prediction process of an operable time by a biological signal measurement device 1 of the second embodiment. The same step numbers are given to the same parts as those in the prediction process of the first embodiment (FIG. 10).

Hereinafter, the process different from that of the first embodiment will be mainly described.

In step S100, the remaining battery level acquisition unit 31 acquires information about a remaining battery level RM of the biological signal measurement device 1. In step S101, the operable time prediction unit 32 reads information about power consumption PC1 in the first operation mode and power consumption PC2 in the second operation mode from the storage unit 25. Up to this point, the process is the same as in the first embodiment.

In step S200, the operable time prediction unit 32 reads the time series data of the ECG signals measured in the past from the storage unit 25, analyzes the state (quality) of the waveform, and divides the time series data into a clean section in which the quality of the waveform is equal to or higher than a predetermined criterion and a noisy section in which the quality of the waveform is lower than the predetermined criterion. This process is the same as the process described in steps S511, S512, S521, S522, and S530 in FIG. 6. As the data to be analyzed, for example, it is advisable to use data from at least the past 24 hours. Since the amount of activity (body movement) during the day and while sleeping is completely different, for example, when the determination is made only based on the data while sleeping, only the clean section is present, and when the determination is made only based on the data during the day, the noisy section may increase. Therefore, by using 24-hour data that reflects the user's life cycle, it is possible to obtain valid results that are not biased by time period.

In step S201, the operable time prediction unit 32 predicts the proportion of the operation time in the first operation mode and the operation time in the second operation mode based on the result obtained in step S200. For example, when a length of the noisy section in the past 24 hours is Tn [h] and a length of the clean section is Tc [h], the operable time prediction unit 32 may calculate a proportion R1 of the future operation time in the first operation mode and a proportion R2 of the future operation time in the second operation mode using the following formulas.

$$R1 = Tn / (Tn + Tc)$$

$$R2 = Tc / (Tn + Tc)$$

In step S202, the operable time prediction unit 32 predicts an operable time AT based on the proportions R1 and R2 of the future operation times in the first operation mode and the second operation mode, the power consumption PC1 in the first operation mode, the power consumption PC2 in the second operation mode, and the remaining battery level RM.

When the biological signal measurement device 1 operates in the first operation mode for a time (R1 × AT) and in the second operation mode for a time (R2 ×

AT), the amount of power consumed in each operation mode is (R1 x AT) x PC1 [W-h] and (R2 x AT) x PC2 [W-h], the sum of these two power modes should be equal to the remaining battery level RM, and (R1 x AT) x PC1 + (R2 x AT) x PC2 = RM. Solving this for AT gives AT [h] = RM [W-h]/(R1 x PC1 [W] + R2 x PC2 [W]). By using this prediction formula, it is possible to predict the operable time AT taking into consideration the operation time proportion in each of the first operation mode and the second operation mode.

In step S203, the output unit 33 displays the information about the predicted value AT on a display, or notifies an external device (such as the user's smartphone) via the communication unit 26.

In the prediction process of the present embodiment described above, in the wearable biological signal measurement device 1 that can dynamically switch the number of channels to be activated, it is possible to predict the operable times AT1 and AT2 taking into account the power consumption PC1 and PC2 for each operation mode and the proportions R1 and R2 of the future operation time, and provide information to inform the user of the appropriate timing for charging. In the present embodiment, the proportions R1 and R2 of the operation time in each operation mode are predicted by analyzing the user's own past measurement data, such that the operable time can be estimated with high accuracy when the user uses the device as usual. This improves the usability of the biological signal measurement device 1 and enables 24-hour monitoring with minimal gaps in measurement.

In the present embodiment, the past measurement data is divided into two sections: a noisy section and a clean section. However, the clean section may be further divided into an abnormal waveform section in which an abnormal waveform appears and a normal waveform section in which no abnormal waveform appears. Specifically, in the process of step S200, the operable time prediction unit 32 evaluates fluctuations in the heart rate interval (RRI) in the clean section, and classifies a section in which the heart rate interval is constant as a "normal waveform section" with no abnormal waveform, and a section in which the heart rate interval fluctuates randomly as an "abnormal waveform section" where an abnormal waveform appears. This process is the same as the process of step S540 in the first embodiment. Then, in step S201, the operable time prediction unit 32 calculates the proportion R1 of the operation time in the first operation mode and the proportion R2 of the operation time in the second operation mode using the following formula. Tca is the length of the normal waveform section, and Tcb is the length of the abnormal waveform section.

$$R1 = (Tn + Tcb) / (Tn + Tc)$$

$$R2 = Tca / (Tn + Tc)$$

$$Tc = Tca + Tcb$$

In other words, the proportion of the sum of the times of the noisy section and the abnormal waveform section in the past 24 hours is the proportion of the operation time in the first operation mode, and the proportion of the time of the normal waveform section in the past 24 hours is the proportion of the operation time in the second operation mode. The prediction formula for the operable time AT may be the same as that described above.

As described with reference to FIG. 9, the biological signal measurement device 1 performs control to switch to the first operation mode in order to detect P waves with high accuracy when an abnormal waveform is detected. Therefore, by taking abnormal waveform sections into consideration in the prediction process, it is expected that a more realistic estimate of the operable time will be possible.

<Third Embodiment>

In the first and second embodiments, the user is notified by displaying the predicted value of the operable time on the display, but it may be possible to further determine whether charging is necessary based on the predicted value, and notify the user that charging is necessary at an appropriate time.

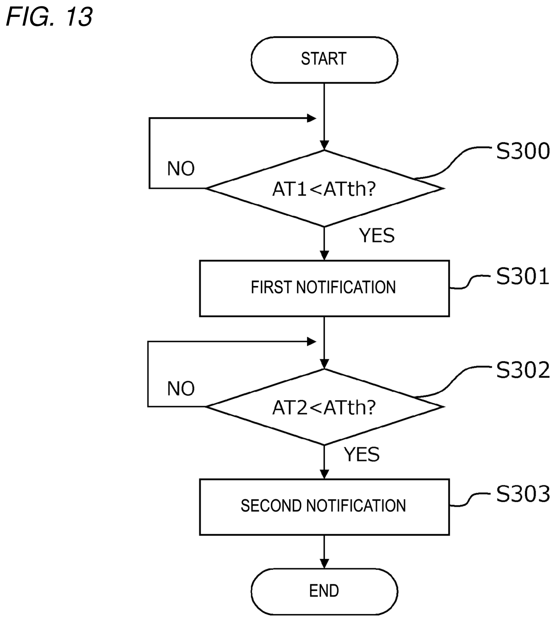

FIG. 13 illustrates an example of a notification process when the minimum value AT1 and maximum value AT2 of the operable time are predicted as in the first embodiment. The output unit (notification unit) 33 compares the minimum value AT1 of the operable time obtained in the most recent prediction process with a threshold value ATth (step S300). When the minimum value AT1 falls below the threshold value ATth (YES in step S300), a first notification is executed (step S301). The notification may be provided by sound, vibration, light, electrical stimulation, or any other means. Then, the output unit (notification unit) 33 compares the maximum value AT2 of the operable time with the threshold value ATth (step S302), and when the maximum value AT2 also falls below the threshold value ATth (YES in step S302), a second notification is executed (step S303). By providing such a notification, the user can be made aware that the time for charging is approaching, and thus can prevent forgetting to charge the battery. In this case, the notification method may be different between the first notification and the second notification, such as by using different sounds or light emitting colors, or by using different light emitting methods or vibration methods. The threshold value ATth may be set arbitrarily, for example, to about one or two hours. The threshold value ATth may be changed by the user.

<Fourth Embodiment>

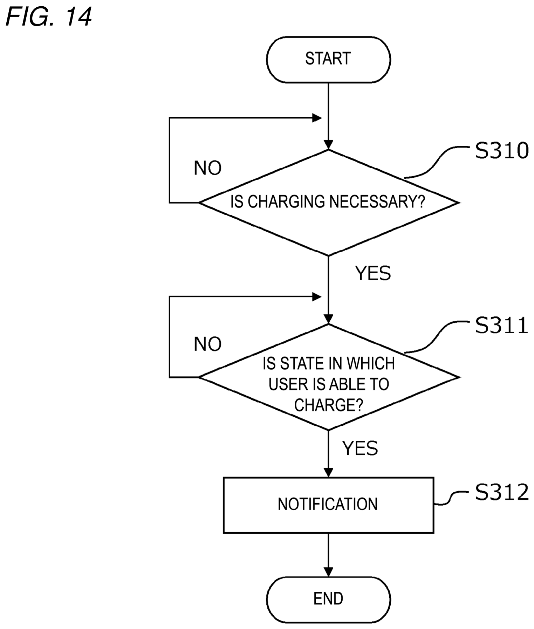

FIG. 14 illustrates an example of a notification process when the predicted value AT of the operable time is calculated as in the second embodiment. The output unit (notification unit) 33 determines whether charging is necessary based on the predicted value AT of the operable time obtained in the most recent prediction process (step S310). The criteria for determining whether it is necessary may be set in any way. For example, it may be determined that "charging is necessary" when the predicted value AT falls below a predetermined threshold value ATth2. The threshold value ATth2 may be set arbitrarily, and may be set to, for example, about 3 to 6 hours with a certain margin. The threshold value ATth2 may be changed by the user.

Even if a notification is provided while the user is out or at work, the user will not be able to charge the battery, and therefore, such a notification is wasteful and troublesome for the user. When it is determined that charging is necessary (YES in step S310), the output unit (notification unit) 33 determines whether the user is in a state in which charging is possible (step S311). Then, when it is determined that the user is in a state in which charging is possible (YES in step S311), the output unit (notification unit) 33 notifies the user (step S312).

The determination as to whether the user is in a state in which charging is possible may be made in any way. For example, in a time period in which the user is at home or in a time period in which the user usually performs charging, it may be considered that the user is highly likely to be in a state in which charging is possible.

For example, the output unit (notification unit) 33 may read information about a preset time of returning home from the storage unit 25, and may consider that the user has returned home (that is, that the user has entered a state in which charging is possible) when the current time passes that time of returning home. The value of the time of returning home may be set by the user, or may be set as a fixed value that is the time an average office worker returns home (for example, 07:00 p.m.).

Alternatively, the biological signal measurement device 1 may be linked to a schedule management service such as Google Calendar. For example, the output unit (notification unit) 33 may acquire the user's schedule information from an external device via the communication unit 26, and determine the user's time of returning and the at-home time period in which the user is at home based on the schedule information.

Alternatively, when the biological signal measurement device 1 records a history of the times when charging was performed in the past in the storage unit 25, a time period in which the user can charge may be estimated based on the charging history information. Many users charge the device 1 in accordance with their own lifestyle (for example, while taking a bath), and therefore charging is often performed at roughly the same time period. Therefore, by referring to the user's own charging history information, it is possible to estimate the optimal charging timing for that user.

Alternatively, when the biological signal measurement device 1 has a built-in positioning sensor such as GPS or is capable of cooperating with an external device that has such a positioning sensor, it may determine whether the user is at home based on the user's current location information.

With any of the above methods, the notification is provided when the user is in a state in which charging is possible (or when there is a high probability that this will be the case), and thus the user can receive an effective reminder at an appropriate timing.

In step S310, it is determined that "charging is necessary" when the predicted value AT of the operable time falls below the threshold value ATth2, but a user's chargeable time period may also be taken into consideration when determining whether charging is necessary. For example, a case is considered where the current time is 4:00 p.m., the predicted value AT of the operable time is 20 hours, the threshold value ATth2 is 3 hours, and the user's chargeable time period is 7:00 p.m. to 11:00 p.m. In this case, in the process of step S310 described above, it is determined that "charging is necessary" when the operable time falls below three hours, that is, around 9:00 a.m. the next day, 17 hours from now. However, even if the notification is provided at that time, the user will not be able to charge the battery. In this case, the device 1 will run out of battery at around 12 o'clock and stop, resulting in a situation where measurements cannot be taken for a long period of time. Therefore, the output unit (notification unit) 33 acquires information about the current time and information about the chargeable time period in which the user is expected to be able to charge the battery the following day, and when the expected operation stop time of the biological signal measurement device 1, which is calculated from the current time and the operable time, arrives before the chargeable time period on the following day, it may determine that charging is necessary during the current day. In the above case, the expected operation stop time is 12 o'clock the next day, which arrives before the chargeable time period of 7:00 p.m. to 11:00 p.m. the next day. Therefore, it is determined that charging is necessary during the current day, and a notification is provided to the user during the chargeable time period during the current day (although there is still sufficient operable time remaining). By performing this type of determination process, the notification is provided ahead of schedule, and thus it is possible to prevent the biological signal measurement device 1 from stopping during the time period in which the user cannot charge the battery. The information about the chargeable time period may be set in advance in the storage unit 25, may be acquired as schedule information from an external device, or may be input by the user.

<Others>

The embodiments described above are merely illustrative of configuration examples of the present invention. The present invention is not limited to the specific aspects described above, and various modifications are possible within the scope of the technical idea of the present invention. For example, the body part on which the biological signal measurement device is attached may be other than the upper arm. As the fixing member for fixing the electrodes 11 in a state in which the electrodes are pressed against the living body, a band-shaped (belt-shaped) member that is wrapped around the measurement portion of the living body in a state in which the electrodes 11 are brought into contact, as in the above embodiments, an envelope-shaped member that covers and wraps the measurement portion of the living body, or a ringshaped member may be used. The fixing member may have elasticity or deformability in order to correspond to the size (diameter) of the measurement portion of the living body. Alternatively, when the fixing member is made of a non-elastic material, the fixing member may have a structure that allows adjustment of the length. The number of channels may be any number equal to or greater than two. The arrangement of the electrodes 11 is not necessarily one line, and the electrodes 11 may be arranged in a two-dimensional array. The electrodes 11 may be arranged at irregular intervals, instead of at equal intervals. The electrode 11 may be integrated with the fixing member (band 10), or may have a separate structure from the fixing member. The control body 12 need not be fixed to the band 10. For example, the control body 12 and the band 10 may be formed as separate structures, and the control body 12 and the band 10 (the electrode 11) may be connected to each other by a cable. The biological signals to be measured are not limited to ECG signals, but may also be, for example, electromyographic signals, electroencephalogram signals, pulse waves, or blood pressure. The configurations of the above embodiments may be combined with each other as long as no technical contradictions arise.

The present specification includes the following disclosures.

[Supplementary Note 1]

A biological signal measurement device (1) that is wearable, capable of measuring biological signals from a plurality of channels, and driven by a battery, the biological signal measurement device (1) including:

an operation mode switching unit (30) configured to switch between a first operation mode in which the plurality of channels are activated and biological signals from the plurality of channels are measured and a second operation mode in which only some of the channels are activated and biological signals from some of the channels are measured;

a remaining battery level acquisition unit (31) configured to acquire remaining battery level information of the battery;

a prediction unit (32) configured to predict an operable time of the biological signal measurement device (1) based on power consumption in each operation mode and the remaining battery level; and

an output unit (33) configured to output information about a predicted value of an operable time.

[Supplementary Note 2]

The biological signal measurement device (1) according to Supplementary Note 1, wherein

the prediction unit (32) predicts a first operable time when the biological signal measurement device continues operating in the first operation mode based on the power consumption in the first operation mode and the remaining battery level and predicts a second operable time when the biological signal measurement device continues operating in the second operation mode based on the power consumption in the second operation mode and the remaining battery level, and

the output unit (33) outputs the first operable time and the second operable time as minimum and maximum values, respectively, of the predicted value of the operable time.

[Supplementary Note 3]

The biological signal measurement device (1) according to Supplementary Note 1 or 2, wherein the output unit (33) determines whether charging is necessary based on the predicted value of the operable time, and notifies a user that charging is necessary when charging is necessary.

[Supplementary Note 4]

The biological signal measurement device (1) according to Supplementary Note 3, wherein the output unit (33) controls a timing of a notification such that the notification is provided during a time period in which the user is able to charge the battery.

[Supplementary Note 5]

The biological signal measurement device (1) according to Supplementary Note 4, wherein the output unit (33) considers that the user has returned home when a current time passes a preset time and provides the notification.

[Supplementary Note 6]

The biological signal measurement device (1) according to Supplementary Note 4, wherein the output unit (33) determines an at-home time period in which the user is at home based on schedule information of the user acquired from an external device and provides the notification during the at-home time period.

[Supplementary Note 7]

The biological signal measurement device (1) according to Supplementary Note 4, wherein the output unit (33) estimates a time period in which the user is able to charge the battery based on charging history information that records a time when charging has been performed in the past, and provides the notification during the time period estimated to be available for charging.

[Supplementary Note 8]

The biological signal measurement device (1) according to Supplementary Note 4, wherein the output unit (33) determines whether the user is at home based on location information of the user, and provides the notification when it is determined that the user is at home.

[Supplementary Note 9]

The biological signal measurement device (1) according to any one of Supplementary Notes 3 to 8, wherein the output unit (33) notifies the user that charging is necessary using at least one of sound, vibration, light, and electrical stimulation.

[Supplementary Note 10]

The biological signal measurement device (1) according to any one of Supplementary Notes 3 to 9, wherein

the output unit (33) includes a notification device (123) configured to notify the user that charging is necessary using light, and
the notification device (123) is disposed at a position visible to the user wearing the biological signal measurement device (1).

[Supplementary Note 11]

The biological signal measurement device (1) according to any one of Supplementary Notes 3 to 10, wherein the output unit (33) acquires information about a current time and information about a chargeable time period in which the user is expected to be able to charge the battery the following day and determines that charging is necessary during the current day when an expected operation stop time of the biological signal measurement device (1), which is calculated from the current time and the operable time, arrives before the chargeable time period on the following day.

[Supplementary Note 12]

The biological signal measurement device (1) according to any one of Supplementary Notes 1 to 11, wherein the prediction unit (32) predicts a proportion of a future operation time in each of the first operation mode and the second operation mode, and calculates the operable time based on the proportion of the future operation time in each operation mode, the power consumption in each operation mode, and the remaining battery level.

[Supplementary Note 13]

The biological signal measurement device (1) according to Supplementary Note 12, wherein the prediction unit (32) analyzes time series data of biological signals measured in the past and predicts a proportion of a future operation time in each operation mode based on a result of an analysis.

[Supplementary Note 14]

The biological signal measurement device (1) according to Supplementary Note 13, wherein

the prediction unit (32)
divides, by analyzing a state of a waveform of the time series data of the biological signals measured in the past, the time series data into a clean section in which a quality of the waveform is equal to or higher than a predetermined criterion and a noisy section in which the quality of the waveform is lower than the predetermined criterion,
predicts a proportion of a future operation time in the first operation mode from a proportion of the noisy section, and
predicts a proportion of a future operation time in the second operation mode from a proportion of the clean section.

[Supplementary Note 15]

The biological signal measurement device (1) according to Supplementary Note 13, wherein

the prediction unit (32)
divides, by analyzing a state of a waveform of the time series data of the biological signals measured in the past, the time series data into a clean section in which a quality of the waveform is equal to or higher than a predetermined criterion and a noisy section in which the quality of the waveform is lower than the predetermined criterion and further divides the clean section into an abnormal waveform section in which an abnormal waveform appears and a normal waveform section in which the abnormal waveform does not appear,

predicts a proportion of a future operation time in the first operation mode from a proportion of the noisy section and the abnormal waveform section, and predicts a proportion of a future operation time in the second operation mode from a proportion of the normal waveform section.

[Supplementary Note 16]

The biological signal measurement device (1) according to any one of Supplementary Notes 1 to 15, wherein the biological signal is an electrocardiogram (ECG) signal.

REFERENCE SIGNS

1: Biological signal measurement device
10: Band
11: Electrode
12: Control body
13: Fixing mechanism

**Claims**

1. A biological signal measurement device that is wearable, capable of measuring biological signals from a plurality of channels, and driven by a battery, the biological signal measurement device comprising:

   an operation mode switching unit configured to switch between a first operation mode in which the plurality of channels are activated and biological signals from the plurality of channels are measured and a second operation mode in which only some of the channels are activated and biological signals from some of the channels are measured;
   a remaining battery level acquisition unit configured to acquire remaining battery level information of the battery;
   a prediction unit configured to predict an operable time of the biological signal measurement device based on power consumption in each operation mode and the remaining battery level; and
   an output unit configured to output information about a predicted value of an operable time.

2. The biological signal measurement device according to claim 1, wherein

   the prediction unit predicts a first operable time when the biological signal measurement device continues operating in the first operation mode based on the power consumption in the first operation mode and the remaining battery level and predicts a second operable time when the biological signal measurement device continues operating in the second operation mode based on the power consumption in the second operation mode and the remaining battery level, and the output unit outputs the first operable time and the second operable time as minimum and maximum values, respectively, of the predicted value of the operable time.

3. The biological signal measurement device according to claim 1, wherein the output unit determines whether charging is necessary based on the predicted value of the operable time and notifies a user that charging is necessary when charging is necessary.

4. The biological signal measurement device according to claim 3, wherein the output unit controls a timing of a notification such that the notification is provided during a time period in which the user is able to charge the battery.

5. The biological signal measurement device according to claim 4, wherein the output unit considers that the user has returned home when a current time passes a preset time and provides the notification.

6. The biological signal measurement device according to claim 4, wherein the output unit determines an at-home time period in which the user is at home based on schedule information of the user acquired from an external device and provides the notification during the at-home time period.

7. The biological signal measurement device according to claim 4, wherein the output unit estimates a time period in which the user is able to charge the battery based on charging history information that records a time when charging has been performed in the past and provides the notification during the time period estimated to be available for charging.

8. The biological signal measurement device according to claim 4, wherein the output unit determines whether the user is at home based on location information of the user and provides the notification when it is determined that the user is at home.

9. The biological signal measurement device according to claim 3, wherein the output unit notifies the user that charging is necessary using at least one of sound, vibration, light, and electrical stimulation.

10. The biological signal measurement device according to claim 3, wherein

the output unit includes a notification device configured to notify the user that charging is necessary using light, and
the notification device is disposed at a position visible to the user wearing the biological signal measurement device.

11. The biological signal measurement device according to claim 3, wherein the output unit acquires information about a current time and information about a chargeable time period in which the user is expected to be able to charge the battery the following day and determines that charging is necessary during the current day when an expected operation stop time of the biological signal measurement device, which is calculated from the current time and the operable time, arrives before the chargeable time period on the following day.

12. The biological signal measurement device according to claim 1, wherein the prediction unit predicts a proportion of a future operation time in each of the first operation mode and the second operation mode and calculates the operable time based on the proportion of the future operation time in each operation mode, the power consumption in each operation mode, and the remaining battery level.

13. The biological signal measurement device according to claim 11, wherein the prediction unit analyzes time series data of biological signals measured in the past and predicts a proportion of a future operation time in each operation mode based on a result of an analysis.

14. The biological signal measurement device according to claim 13, wherein

the prediction unit
divides, by analyzing a state of a waveform of the time series data of the biological signals measured in the past, the time series data into a clean section in which a quality of the waveform is equal to or higher than a predetermined criterion and a noisy section in which the quality of the waveform is lower than the predetermined criterion,
predicts a proportion of a future operation time in the first operation mode from a proportion of the noisy section, and
predicts a proportion of a future operation time in the second operation mode from a proportion of the clean section.

15. The biological signal measurement device according to claim 13, wherein

the prediction unit

divides, by analyzing a state of a waveform of the time series data of the biological signals measured in the past, the time series data into a clean section in which a quality of the waveform is equal to or higher than a predetermined criterion and a noisy section in which the quality of the waveform is lower than the predetermined criterion and further divides the clean section into an abnormal waveform section in which an abnormal waveform appears and a normal waveform section in which the abnormal waveform does not appear,
predicts a proportion of a future operation time in the first operation mode from a proportion of the noisy section and the abnormal waveform section, and
predicts a proportion of a future operation time in the second operation mode from a proportion of the normal waveform section.

16. The biological signal measurement device according to any one of claims 1 to 15, wherein the biological signal is an electrocardiogram (ECG) signal.

# FIG. 1

**PREDICTION OF OPERABLE TIME IN CONSIDERATION OF DYNAMIC SWITCHING OF NUMBER OF ACTIVE CHANNELS**

10

1

**DETERMINATION OF NECESSITY OF CHARGING BASED ON PREDICTED VALUE**

**NOTIFICATION OF CHARGING TIMING**

BEEP!

BEEP!

EP 4 736 767 A1

FIG. 2

FIG. 3

FIG. 4

FIG. 5

EP 4 736 767 A1

## FIG. 6

*FIG. 7*

FIRST OPERATION MODE (MULTICHANNEL)

DYNAMIC SWITCHING

SECOND OPERATION MODE (SINGLE CHANNEL)

FIG. 8

FIG. 9

DISTURBANCE IN HEART RATE INTERVAL

ECG

SIGNAL QUALITY

CLEAN — NOISY — CLEAN

(NORMAL WAVEFORM) (NORMAL WAVEFORM) (ABNORMAL WAVEFORM) (NORMAL WAVEFORM)

OPERATION MODE

| SECOND | FIRST | SECOND | FIRST | SECOND |

POWER CONSUMPTION

HIGH

LOW

EP 4 736 767 A1

## FIG. 10

```
          ┌──────────────┐
          │    START     │
          └──────────────┘
                 │
                 ▼
      ┌─────────────────────┐
      │ ACQUIRE INFORMATION │ ── S100
      │   ABOUT REMAINING   │
      │    BATTERY LEVEL    │
      └─────────────────────┘
                 │
                 ▼
      ┌─────────────────────┐
      │ ACQUIRE INFORMATION │ ── S101
      │  ABOUT POWER        │
      │  CONSUMPTION IN EACH│
      │   OPERATION MODE    │
      └─────────────────────┘
                 │
                 ▼
      ┌─────────────────────┐
      │  PREDICT OPERABLE   │ ── S102
      │  TIME IN FIRST      │
      │  OPERATION MODE     │
      └─────────────────────┘
                 │
                 ▼
      ┌─────────────────────┐
      │  PREDICT OPERABLE   │ ── S103
      │  TIME IN SECOND     │
      │  OPERATION MODE     │
      └─────────────────────┘
                 │
                 ▼
      ┌─────────────────────┐
      │  OUTPUT PREDICTED   │ ── S104
      │  VALUE OF OPERABLE  │
      │       TIME          │
      └─────────────────────┘
                 │
                 ▼
          ┌──────────────┐
          │     END      │
          └──────────────┘
```

## FIG. 11

OPERABLE TIME:
    MINIMUM OF 8 HOURS TO MAXIMUM OF 16 HOURS


BATTERY MAY RUN OUT BETWEEN 8:00 P.M.
TODAY AND 4:00 A.M. TOMORROW
PLANNED CHARGING IS RECOMMENDED


                CURRENT TIME 12:00

FIG. 12

```
        ┌─────────────┐
        │    START    │
        └──────┬──────┘
               │
               ▼
    ┌─────────────────────┐
    │ ACQUIRE INFORMATION │      S100
    │  ABOUT REMAINING    │
    │   BATTERY LEVEL     │
    └──────────┬──────────┘
               │
               ▼
    ┌─────────────────────┐
    │ ACQUIRE INFORMATION ABOUT │  S101
    │ POWER CONSUMPTION IN EACH  │
    │    OPERATION MODE     │
    └──────────┬──────────┘
               │
               ▼
    ┌─────────────────────┐
    │    ANALYZE PAST     │      S200
    │ MEASUREMENT DATA AND │
    │  DIVIDE DATA INTO CLEAN │
    │ SECTION AND NOISY SECTION │
    └──────────┬──────────┘
               │
               ▼
    ┌─────────────────────┐
    │ PREDICT PROPORTION OF │    S201
    │  OPERATION TIME IN EACH │
    │    OPERATION MODE    │
    └──────────┬──────────┘
               │
               ▼
    ┌─────────────────────┐
    │ PREDICT OPERABLE TIME │    S202
    └──────────┬──────────┘
               │
               ▼
    ┌─────────────────────┐
    │ OUTPUT PREDICTED VALUE │   S203
    │   OF OPERABLE TIME   │
    └──────────┬──────────┘
               │
               ▼
        ┌─────────────┐
        │     END     │
        └─────────────┘
```

*FIG. 13*

```
                    ┌─────────────┐
                    │    START    │
                    └─────────────┘
                           │
                           ▼
        ┌──►         ◇─────────────◇              S300
        │      NO   ◇  AT1<ATth?  ◇
        │           ◇─────────────◇
        │                  │ YES
        │                  ▼
        │      ┌───────────────────────┐         S301
        │      │   FIRST NOTIFICATION  │
        │      └───────────────────────┘
        │                  │
        │                  ▼
        ┌──►         ◇─────────────◇              S302
        │      NO   ◇  AT2<ATth?  ◇
        │           ◇─────────────◇
        │                  │ YES
        │                  ▼
        │      ┌───────────────────────┐         S303
        │      │  SECOND NOTIFICATION  │
        │      └───────────────────────┘
        │                  │
                           ▼
                    ┌─────────────┐
                    │     END     │
                    └─────────────┘
```

FIG. 14

## INTERNATIONAL SEARCH REPORT

| International application No. |
|---|
| **PCT/JP2024/028011** |

| A. | CLASSIFICATION OF SUBJECT MATTER |
|---|---|

*A61B 5/332*(2021.01)i; *A61B 5/256*(2021.01)i; *A61B 5/352*(2021.01)i; *A61B 5/353*(2021.01)i
FI: A61B5/332; A61B5/256 220; A61B5/352 100; A61B5/353

According to International Patent Classification (IPC) or to both national classification and IPC

| B. | FIELDS SEARCHED |
|---|---|

Minimum documentation searched (classification system followed by classification symbols)

A61B5/24-5/398

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2020/0069953 A1 (WEST AFFUM HOLDINGS CORP.) 05 March 2020 (2020-03-05) paragraphs [0083], [0094], [0097], [0101], fig. 1-5 | 1-16 |
| A | CN 107049301 A (DONGGUAN MATHEMATICAL ENGINEERING ACADEMY OF CHINESE MEDICINE, GUANGZHOU UNIVERSITY OF CHINESE MEDICINE) 18 August 2017 (2017-08-18) paragraph [0036], fig. 1-2 | 1-16 |
| A | WO 2017/122379 A1 (OSAKA UNIVERSITY) 20 July 2017 (2017-07-20) paragraph [0096] | 1-16 |
| A | US 5289824 A (INSTROMEDIX, INC.) 01 March 1994 (1994-03-01) column 9, line 57 to column 12, line 21, fig. 1 | 1-16 |
| A | JP 2007-517553 A (COMPUMEDICS LTD.) 05 July 2007 (2007-07-05) paragraph [0116] | 1-16 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 October 2024** | **15 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2024/028011** |

**C.     DOCUMENTS CONSIDERED TO BE RELEVANT**

| Category* | Citation of document, with indication, where appropriate, of the relevant passages | Relevant to claim No. |
| --- | --- | --- |
| A | US 2010/0114204 A1 (MEDTRONIC, INC.) 06 May 2010 (2010-05-06) paragraph [0132] | 1-16 |

Form PCT/ISA/210 (second sheet) (July 2022)

## INTERNATIONAL SEARCH REPORT
### Information on patent family members

International application No.

**PCT/JP2024/028011**

| Patent document cited in search report | | | Publication date (day/month/year) | Patent family member(s) | | | Publication date (day/month/year) |
|---|---|---|---|---|---|---|---|
| US | 2020/0069953 | A1 | 05 March 2020 | (Family: none) | | | |
| CN | 107049301 | A | 18 August 2017 | (Family: none) | | | |
| WO | 2017/122379 | A1 | 20 July 2017 | US | 2019/0029595 | A1 | |
| | | | | paragraph [0105] | | | |
| | | | | EP | 3403576 | A1 | |
| US | 5289824 | A | 01 March 1994 | (Family: none) | | | |
| JP | 2007-517553 | A | 05 July 2007 | US | 2007/0032733 | A1 | |
| | | | | paragraphs [0460], [0462] | | | |
| | | | | WO | 2005/067790 | A1 | |
| US | 2010/0114204 | A1 | 06 May 2010 | WO | 2010/051388 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005027720 A